# DEMANDE DE BREVET EUROPEEN

(11) **EP 1 247 544 A1**
(43) Date de publication de la demande: **09.10.2002**
(21) Numéro de dépôt: 02290836.2
(22) Date de dépôt: 05.04.2002
(51) Int. Cl.: A61N 1/37

(54) **Procédé de traitement et de mémorisation sous forme différenciée de données relatives à des fonctions Holter, notamment pour un dispositif médical implantable actif**

(30) Priorité: 06.04.2001 FR 0104660
(71) Demandeur: ELA MEDICAL, 92541 Montrouge (FR)
(72) Inventeur: Graindorge, Laurence, 92290 Chatenay Malabry (FR)
(74) Mandataire: Dupuis-Latour, Dominique

(57) **Abrégé**

Ce procédé comprend les étapes consistant à :
a) mémoriser dans un premier secteur mémoire (10) une représentation détaillée des données Holter (14, 16, 18, 20) sur des intervalles successifs courts (22), notamment horaires, ces intervalles courts se succédant sur une période courte, notamment journalière ;
b) périodiquement, lire dans le premier secteur mémoire la représentation détaillée et traiter les données de manière à élaborer sur un intervalle long (24), notamment journalier, une représentation consolidée des données sur ladite période courte ;
c) mémoriser cumulativement dans un second secteur mémoire (12) la représentation consolidée, pour donner une représentation des données sur une série d'intervalles longs (24) se succédant sur une période longue, notamment plurihebdomadaire ou plurimensuelle ;
d) libérer le premier secteur mémoire pour permettre d'y mémoriser une représentation détaillée de nouvelle données.

## Description

L'invention concerne les "dispositifs médicaux actifs", notamment les "dispositifs médicaux implantables actifs" tels que définis par la Directive 90/385/CE du 20 juin 1990 du Conseil des communautés européennes. Cette définition inclut les stimulateurs cardiaques, défibrillateurs, cardio-verteurs et/ou dispositifs multisite, mais aussi les appareils neurologiques, les pompes de diffusion de substances médicales, les implants cochléaires, les capteurs biologiques implantés, etc., ainsi que les dispositifs de mesure de pH ou encore de mesure d'impédance intracorporelle (telle que la mesure de l'impédance transpulmonaire ou de l'impédance intracardiaque).

L'invention concerne plus précisément les fonctions de traitement et d'enregistrement de données appelées "données Holter", par exemple relatives à l'activité cardiaque, enregistrées sur une longue période, de plusieurs jours à plusieurs mois.

Dans la présente description on se référera principalement aux fonctions Holter gérées par des appareils implantés tels que stimulateurs, cardio-verteurs ou défibrillateurs, notamment des appareils implantés qu'il est possible d'interroger ultérieurement par télémétrie au moyen d'un appareil externe ou "programmateur".

L'invention peut cependant être transposée au cas des appareils Holter simples, externes, ayant pour seule fonction la surveillance et l'enregistrement de l'activité cardiaque.

De la même façon, bien que l'on se référera principalement aux signaux cardiaques recueillis au moyen d'électrodes implantées, les fonctions mises en oeuvre par l'invention peuvent également être appliquées à des signaux recueillis par des électrodes externes, ainsi qu'à des compteurs d'événements ou à des signaux non recueillis mais représentatifs d'un état ou d'une action de l'implant, par exemple l'application d'une thérapie de choc, la mesure d'une impédance de sonde, etc.

Le stockage de ces diverses données peut être effectué de diverses manières.

Une première technique, dite cumulative, consiste à conserver les données de façon indifférenciée sur la globalité de la période (typiquement, sur les six mois de la période de suivi), en horodatant chaque événement au moyen d'un marqueur qui servira de repère temporel lorsque les données seront traitées après avoir été lues par le programmateur, de manière à reconstituer la chronologie des différents événements successifs.

Cette technique est économe en termes de capacité mémoire requise, mais elle est limitée à la mémorisation d'événements ponctuels et ne permet pas de suivre des paramètres variant de façon continue, par exemple pour étudier l'évolution de la fréquence cardiaque moyenne, d'une impédance, de l'index d'apnée, etc.

Une autre technique consiste à attribuer un champ mémoire à chaque intervalle de temps (typiquement d'une journée), où sont mémorisées les données correspondant à chacune de ces périodes, notamment l'évolution de paramètres variant de façon continue. En attribuant un champ mémoire pour les données recueillies au cours d'une même journée, on peut ainsi stocker ces dernières sur une période pouvant aller typiquement jusqu'à six mois, jour par jour.

Après lecture des données par le programmateur, il est possible d'effectuer un certain nombre de traitements informatiques, de nature statistique ou autre, traitements relativement complets dans la mesure où les données ont été conservées identiquement sur toute la durée de la période de six mois.

En revanche, cette technique requiert des ressources mémoire importantes du fait du très grand nombre de champs mémoire qui serait nécessaire, par exemple environ 6 x 30= 180 champs mémoire pour un historique sur six mois où chaque emplacement stockerait les données recueillies sur 24 heures. Un compromis doit donc être fait entre la période totale de couverture et la précision des données mémorisées à l'intérieur de chaque période de temps : si l'on veut un suivi précis, on peut par exemple enregistrer des périodes de 2 jours, mais l'ensemble de l'enregistrement ne couvrira qu'un mois (14 x 2 jours) ; si l'on veut en revanche couvrir une période de temps plus importante, par exemple six mois, il faudra choisir des tranches de temps moins précises, par exemple d'une semaine.

L'invention propose un procédé de traitement et de mémorisation de données Holter permettant de pallier ces divers inconvénients, notamment en optimisant l'occupation de la mémoire, paramètre particulièrement contraignant s'agissant d'un dispositif miniaturisé et implanté.

Essentiellement, l'invention propose de réaliser un stockage différencié des données à court terme et à long terme. Les données à court terme sont mémorisées sur une période courte avec une résolution élevée (par exemple sur un ou deux jours par tranches d'une heure), tandis que les données à long terme sont mémorisées sur une période longue avec une résolution moindre (par exemple sur six mois par tranche d'un jour).

L'un des points de départ de l'invention est en effet la constatation que, lorsque les données Holter sont analysées, la précision des données n'est importante que sur les quelques heures qui précèdent, alors que sur les semaines ou les mois qui précèdent c'est surtout la tendance générale et l'évolution de tel ou tel paramètre qu'il est important d'évaluer. Par exemple, si un diagnostic est effectué à la suite d'un choc de défibrillation appliqué par l'appareil, il sera essentiel d'avoir une image fine des événements survenus lors des dernières 24 ou 48 heures précédant le choc, notamment de leur chronologie, tandis que pour la période plus ancienne une analyse de tendance sera généralement suffisante.

En effet, il est important d'avoir un suivi plus important sur les moments précédant la consultation car : (i) si le patient consulte pour un symptôme ou un événement indésirable gênant, il est fort probable que cet événement soit récent, et (ii) lors de l'interrogation du patient, celui-ci donnera des indications plus précises sur les 1 ou 2 jours qui ont précédé que sur ce qui a pu s'être passé il y a 3 ou 4 mois - en d'autres termes, la précision de l'enregistrement suit la précision du souvenir du patient.

Ce stockage différencié implique une mise à jour périodique de la mémoire, par exemple quotidiennement. Ainsi, par exemple, les données sont stockées par tranche d'une heure sur une journée, et tous les jours ces données sont consolidées, c'est-à-dire condensées sur une tranche d'une journée. De cette manière, on obtient deux enregistrements glissants et imbriqués (à court terme et à long terme), qui permettent d'avoir un suivi à long terme condensé donnant les tendances générales, et un suivi à court terme plus précis sur les événements les plus récents.

Plus précisément, l'invention propose un procédé caractérisé par les étapes consistant à :
a) mémoriser dans un premier secteur mémoire une représentation détaillée desdites données sur des intervalles successifs courts, notamment des intervalles horaires, ces intervalles courts se succédant sur une période courte donnée, notamment une période journalière ;
b) périodiquement, lire dans le premier secteur mémoire la représentation détaillée des données et traiter ces données de manière à élaborer sur un intervalle long, notamment un intervalle journalier, une représentation consolidée des données sur ladite période courte ;
c) mémoriser cumulativement dans un second secteur mémoire la représentation consolidée ainsi élaborée, pour donner une représentation des données sur une série d'intervalles longs se succédant sur une période longue donnée, notamment une période plurihebdomadaire ou plurimensuelle ;
d) libérer le premier secteur mémoire pour permettre d'y mémoriser une représentation détaillée de nouvelle données.

Les étapes b) à d) peuvent notamment être exécutées à la fin de chaque période courte, la représentation consolidée de l'étape b) étant élaborée à partir des données de l'avant-dernière période, de sorte que la représentation détaillée sur la dernière période reste conservée en mémoire.

La représentation détaillée des données peut notamment comprendre :
- un histogramme de valeurs établi sur les intervalles courts successifs, le traitement de l'étape b) comprenant l'addition des valeurs de cette histogramme ;
- des valeurs d'un paramètre mesurées au cours de chacun des intervalles courts successifs, le traitement de l'étape b) comprenant le calcul d'une valeur moyenne de ces valeurs mesurées, ou encore la sélection de la valeur minimum et/ou de la valeur maximum atteinte par ces valeurs mesurées ;
- un indicateur de survenue d'un événement, ou de présence d'un état, au cours de chacun des intervalles courts successifs, le traitement de l'étape b) comprenant le positionnement d'un marqueur en cas de présence d'au moins l'un desdits indicateurs au cours de la période courte, ou encore la détermination du nombre d'occurrences dudit indicateur au cours de la période courte.

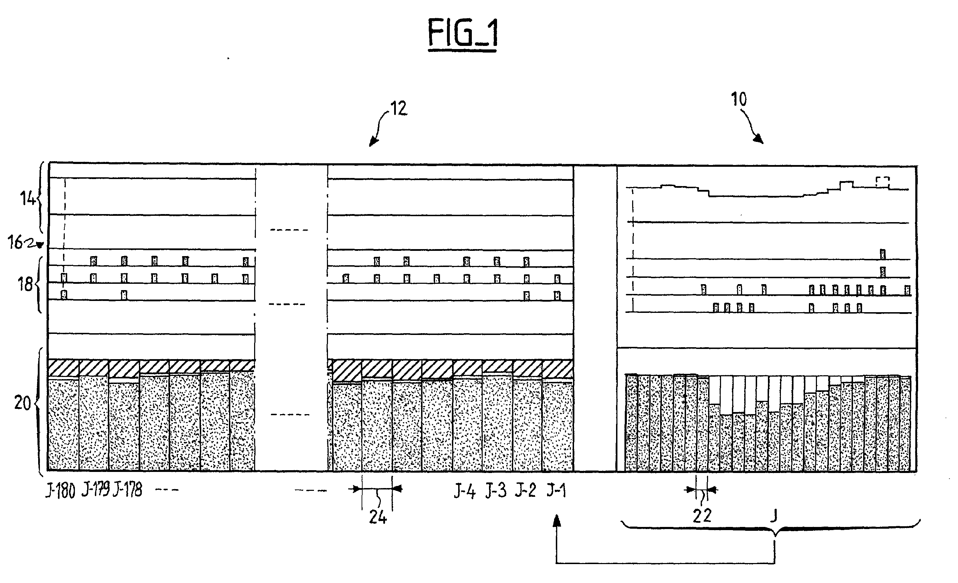

On va maintenant décrire un exemple de mise en oeuvre de l'invention, en référence à la figure unique annexée qui montre sous forme de chronogramme les diverses données stockées en mémoire.

Essentiellement, les données sont stockées sous forme de deux enregistrements distincts, à savoir un enregistrement 10 contenant les données du dernier jour écoulé, avec une résolution horaire, et un enregistrement 12 contenant les données, par exemple des 180 derniers jours écoulés (six mois environ), avec une résolution d'une journée.

Ces deux enregistrements, avec un niveau de détail différent, permettent d'avoir un suivi général à long terme (enregistrement 12, sur six mois) complété par un suivi fin à court terme sur les événements les plus récents (enregistrement 10, sur 24 heures).

En pratique, les informations sont stockées par tranche d'une heure sur une journée. Quotidiennement, c'est à dire lorsque l'on a complété un enregistrement journalier de même type que l'enregistrement 10, ces nouvelles données viennent remplacer le précédent enregistrement journalier 10 qui est, quant à lui, converti en une rubrique de résolution journalière, de l'enregistrement 12 et ajouté à ce dernier (dont le dernier enregistrement devient chronologiquement l'avant dernier, etc.).

Les données peuvent être de plusieurs types :
- courbe, telle que 14, donnant le niveau moyen de la fréquence cardiaque de base, la fréquence des extrasystoles, l'impédance auriculaire, l'impédance ventriculaire, etc.,
- marqueur ou indicateur, tel que 16 ou 18, indiquant la survenue ou non d'un événement donné dans la période considérée, par exemple la survenu d'une extrasystole, le basculement de l'implant dans un mode de fonctionnement particulier, l'application d'une thérapie de choc, etc. ; en variante, au lieu d'un simple indicateur présence/absence de l'événement, il est possible de mémoriser le nombre d'occurrences de l'événement au cours de la période considérée,
- histogramme, tels que 20, donnant par exemple le ratio activité/sommeil, l'indice d'apnée, etc.

Selon le type d'information stockée et son importance, on peut choisir plusieurs modes pour passer d'une tranche courte 22 (résolution horaire) à une tranche longue 24 (résolution journalière) :
- lorsqu'il s'agit de courbes, selon le cas, on peut prendre la moyenne des 24 valeurs pour n'obtenir qu'une valeur sur un jour ; en variante ou en complément, il est également possible de conserver la valeur maximale et/ou minimale atteinte par le paramètre en question sur les 24 heures.
- lorsqu'il s'agit de marqueurs, on peut conserver le nombre d'occurrences sur les 24 heures, ou encore un marqueur indiquant l'existence d'au moins une occurrence sur les 24 heures.
- lorsqu'il s'agit d'histogrammes de valeurs, les 24 histogrammes d'une heure peuvent être sommés pour obtenir un histogramme sur un jour.

## Revendications

1. Un procédé de traitement et de mémorisation de données médicales sur une longue période, notamment de données relatives à des fonctions Holter implantables mises en oeuvre par un dispositif médical implantable actif tel qu'un stimulateur cardiaque, défibrillateur, cardioverteur et/ou dispositif multisite, **caractérisé par** les étapes consistant à :
a) mémoriser dans un premier secteur mémoire (10) une représentation détaillée desdites données (14, 16, 18, 20) sur des intervalles successifs courts (22), notamment des intervalles horaires, ces intervalles courts se succédant sur une période courte donnée, notamment une période journalière ;
b) périodiquement, lire dans le premier secteur mémoire la représentation détaillée des données et traiter ces données de manière à élaborer sur un intervalle long (24), notamment un intervalle journalier, une représentation consolidée des données sur ladite période courte ;
c) mémoriser cumulativement dans un second secteur mémoire (12) la représentation consolidée ainsi élaborée, pour donner une représentation des données sur une série d'intervalles longs (24) se succédant sur une période longue donnée, notamment une période plurihebdomadaire ou plurimensuelle ;
d) libérer le premier secteur mémoire pour permettre d'y mémoriser une représentation détaillée de nouvelle données.

2. Le procédé de la revendication 1, dans lequel les étapes b) à d) sont exécutées à la fin de chaque période courte, la représentation consolidée de l'étape b) étant élaborée à partir des données de l'avant-dernière période, de sorte que la représentation détaillée sur la dernière période reste conservée en mémoire.

3. Le procédé de la revendication 1, dans lequel la représentation détaillée des données comprend un histogramme de valeurs (20) établi sur les intervalles courts successifs, et le traitement de l'étape b) comprend l'addition des valeurs de cette histogramme.

4. Le procédé de la revendication 1, dans lequel la représentation détaillée des données comprend des valeurs d'un paramètre (14) mesurées au cours de chacun des intervalles courts successifs, et le traitement de l'étape b) comprend le calcul d'une valeur moyenne de ces valeurs mesurées.

5. Le procédé de la revendication 1, dans lequel la représentation détaillée des données comprend des valeurs d'un paramètre (14) mesurées au cours de chacun des intervalles courts successifs, et le traitement de l'étape b) comprend la sélection de la valeur minimum et/ou de la valeur maximum atteinte par ces valeurs mesurées.

6. Le procédé de la revendication 1, dans lequel la représentation détaillée des données comprend un indicateur (16, 18) de survenue d'un événement, ou de présence d'un état, au cours de chacun des intervalles courts successifs, et le traitement de l'étape b) comprend le positionnement d'un marqueur en cas de présence d'au moins l'un desdits indicateurs au cours de la période courte.

7. Le procédé de la revendication 1, dans lequel la représentation détaillée des données comprend un indicateur (16, 18) de survenue d'un événement, ou de présence d'un état, au cours de chacun des intervalles courts successifs, et le traitement de l'étape b) comprend la détermination du nombre d'occurrences dudit indicateur au cours de la période courte.
